# EUROPÄISCHE PATENTANMELDUNG

(11) **EP 2 028 189 A2**
(43) Veröffentlichungstag der Anmeldung: **25.02.2009**
(21) Anmeldenummer: 08014111.2
(22) Anmeldetag: 11.07.2005
(51) Int. Cl.: C07K 14/415

(54) **Varianten der Gruppe 1-Allergene aus Poaceae mit reduzierter Allergenität und erhaltener T-Zellreaktivität**

(30) Priorität: 21.07.2004 DE 102004035337
(62) Teilanmeldung aus: 05761824.1
(71) Anmelder: Merck Patent GmbH, 64293 Darmstadt (DE)
(72) Erfinder: Fiebig, Helmut, Prof., 21493 Schwarzenbek (DE); Wald, Martin, Dr., 20253 Hamburg (DE); Nandy, Andreas, Dr., 22175 Hamburg (DE); Kahlert, Helga, Dr., 22041 Hamburg (DE); Weber, Bernhard, Dr., 21031 Hamburg (DE); Cromwell, Oliver, Dr., 23701 Suesel-Fassendorf (DE)

(57) **Zusammenfassung**

Die vorliegende Erfindung betrifft die Herstellung und Verwendung von Varianten der Gruppe-1-Allergene der Poaceae (Süßgräser), welche durch eine gegenüber den bekannten Wildtyp-Allergenen verringerte IgE-Reaktivität und gleichzeitig eine weitgehend erhaltene Reaktivität mit T-Lymphozyten gekennzeichnet sind.

Diese hypoallergenen Allergenvarianten können zur spezifischen Immun-therapie (Hyposensibilisierung) von Patienten mit Gräserpollenallergie oder zur präventiven Immuntherapie von Gräserpollenallergien eingesetzt werden.

## Beschreibung

Die vorliegende Erfindung betrifft die Herstellung und Verwendung von Varianten der Gruppe-1-Allergene der *Poaceae* (Süßgräser), welche durch eine gegenüber den bekannten Wildtyp-Allergenen verringerte IgE-Reaktivität und gleichzeitig eine weitgehend erhaltene Reaktivität mit T-Lymphozyten gekennzeichnet sind.
Diese hypoallergenen Allergenvarianten können zur spezifischen Immuntherapie (Hyposensibilisierung) von Patienten mit Gräserpollenallergie oder zur präventiven Immuntherapie von Gräserpollenallergien eingesetzt werden.
Eine bevorzugte Ausführungsform der Erfindung betrifft Varianten des Hauptallergens Phl p 1 aus dem Pollen von Wiesenlieschgras (*Phleum pratense*)*.*

### Hintergrund der Erfindung

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen (Freidhoff et al., 1986, J. Allergy Clin. Immunol. 78: 1190-2002).
Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.
In Abhängigkeit von der relativen Häufigkeit mit der die einzelnen Allergenmoleküle mit den IgE-Antikörpern von Allergikern reagieren, wird zwischen Major- und Minorallergenen unterschieden.
Im Fall von Wiesenlieschgras (*Phleum pratense*) sind bislang Phl p 1 (Petersen et al., 1993, J. Allergy Clin. Immunol. 92: 789-796), Phl p 5 (Matthiesen und Löwenstein, 1991, Clin. Exp. Allergy 21: 297-307; Petersen et al., 1992, Int. Arch. Allergy Immunol. 98: 105-109), Phl p 6 (Petersen et al., 1995, Int. Arch. Allergy Immunol. 108, 49-54). Phl p 2/3 (Dolecek et al., 1993, FEBS 335 (3), 299-304), Phl p 4 (Haavik et al., 1985, Int. Arch. Allergy Appl. Immunol. 78: 260-268; Valenta et al., 1992, Int. Arch. Allergy Immunol. 97: 287-294, Fischer et al., 1996, J. Allergy Clin. Immunol. 98: 189-198) und Phl p 13 (Suck et al., 2000, Clin. Exp. Allergy 30: 324-332; Suck et al., 2000, Clin. Exp. Allergy 30: 1395-1402) als Majorallergene identifiziert worden.

Die dominierenden Majorallergene des Wiesenlieschgrases (*Phleum pratense*) sind Phl p 1 und Phl p 5. Da die Majorallergene der Gräser der Familie Poaceae untereinander hoch homolog sind und infolge dessen sehr ähnliche biochemische und immunologische Eigenschaften besitzen, werden diese verwandten Proteine als Gruppe 1- bzw. Gruppe 5-Allergene zusammengefaßt.
Die Gruppe 1-Allergene reagieren mit den IgE-Antikörpern von mehr als 95 % der Gräserpollenallergiker und sind somit dominierende Majorallergene der Gräserpollen.
Die Gruppe 1-Allergene sind Glykoproteine mit einer Molekularmasse von ca. 32 kDa und sind im Zytoplasma der Pollenkörner lokalisiert. Sowohl der Kontakt der Pollenkörner mit der Schleimhaut der oberen Atmungswege als auch die Befeuchtung der Pollenkörner durch Regen führen zu einer raschen Freisetzung dieser Allergene. Die rasche Freisetzung der Gruppe 1-Allergene, auch in Form von subzellulären Mikropartikeln, ermöglicht das Eindringen in die unteren Luftwege, was zur Auslösung von schweren Asthmaanfällen führen kann.

Die cDNA's der Gruppe 1-Allergene von *Phleum pratense* (Laffer et al., 1994, J. Allergy Clin. Immunol. 94: 689-698), *Lolium perenne* (Perez et al., 1990, J. Biol. Chem. 265: 16210-16250), *Holcus lanatus* (Schramm et al., 1997, J. Allergy Clin. Immunol. 1999:781-787), *Poa pratensis* (Sturaro u. Viotti, 1998, NCBI GenBank, Acc. No. AJ 131850), *Cynodon dactylon* (Smith et al., 1996, J. Allergy Clin. Immunol. 98: 331-343), *Phalaris aquatica* (Suphioglu et al., 1995, Clin. Exp. Allergy 25 : 853-865) und *Oryza sativa* (Xu et al., 1995, Gene 164: 255-259) sind identifiziert worden.

Außer diesen Erstbeschreibungen der Sequenz sind in Datenbanken weitere Gruppe1-Allergen-Sequenzen publiziert, die in einzelnen Positionen von den originären Sequenzen abweichen. Solche lsoformen sind auch von anderen Gräserpollenallergenen bekannt.

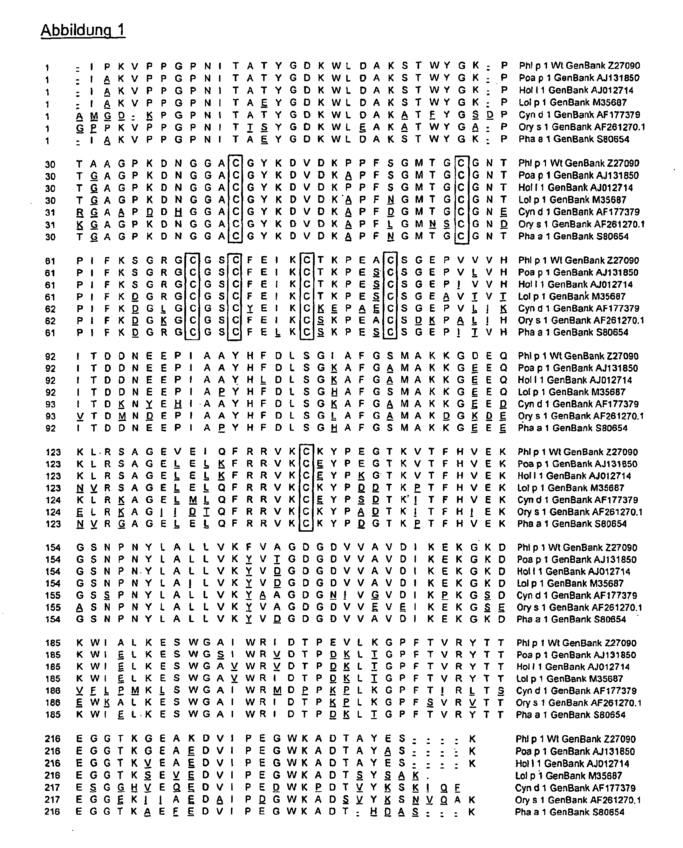

Die Gruppe 1-Allergene der Süßgräser (Poaceae) weisen aufgrund ihrer Homologie eine hohe Kreuzreaktivität mit humanen IgE-Antikörpern auf (Laffer et al., 1996, Mol. Immunology 33: 417-426). Diese immunologische Kreuzreaktivität basiert auf einer sehr ähnlichen Aminosäuresequenz, wie ein Sequenzvergleich von Phl p 1, dem Gruppe 1-Allergen des Wiesenlieschgrases (*Phleum pratense),* mit Gruppe 1-Molekülen von ausgewählten Spezies in Abbildung 1 zeigt.

Sowohl für die Sequenzbereiche der hier bei der Konstruktion hypoallergener Varianten beschriebenen Deletionen der Phl p 1-Aminosäuresequenz als auch für deren flankierenden Sequenzbereiche existieren homologe Sequenzbereiche in den übrigen Gruppe 1-Allergenen der Poaceae. Weiterhin sind sowohl die Anzahl als auch die umgebenden Sequenzbereiche der Cysteine der Gruppe 1-Allergene der *Poaceae* konserviert. Aufgrund von Sequenzhomologien werden die Gruppe 1-Allergene der *Poaceae* der Proteinfamile der β-Expansine zugeordnet (Cosgrove D.J., 2000 Nature 407: 321-6).

Ein klassischer Ansatz zur wirksamen therapeutischen Behandlung von Allergien stellt die spezifische Immuntherapie oder Hyposensibilisierung dar (Fiebig, 1995, Allergo J. 4 (6): 336-339: Bousquet et al., 1998, J. Allergy Clin. Immunol. 102 (4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen. Diese T-Zell-Epitope sind für die therapeutische Wirkung der Allergenpräparate bei der Hyposensibilisierung von entscheidender Bedeutung (Fiebig, 1995, Allergo J. 4 (7): 377-382).

Eine weitergehende Therapieoptimierung wäre mit rekombinant hergestellten Allergenen möglich. Definierte, ggf. auf die individuellen Sensibilierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.

Realistische Perspektiven, die zu einer sicheren Hyposensibilierung mit rekombinanten Expressionsprodukten führen können, bieten gezielt mutierte rekombinante Allergene, bei denen IgE-Epitope spezifisch deletiert werden, ohne die für die Therapie essentiellen T-Zell-Epitope zu beeinträchtigen (Schramm et al., 1999, J. Immunol. 162: 2406-2414).

Ein davon abweichendes Konzept für eine Hyposensibilisierung geht davon aus, dass eine protektive Immunantwort besonders durch blockierende IgG4-Antikörper induziert wird. Entsprechend dieser Hypothese sind rekombinante Phl p 1-Fragmente beschrieben worden, die zur Induktion einer protektiven IgG4-Antwort geeignet sein sollen (Ball et al., 1999, FASEB J. 13:1277-1290). Dieses Konzept unterscheidet sich vollständig von dem Konzept der hypoallergenen Allergenvarianten mit verringerter IgE-Reaktivität und erhaltener T-Zell-Reaktivität.

Eine weitere Möglichkeit zur therapeutischen Beeinflussung des gestörten T-Helferzellen-Gleichgewichtes bei Allergikern ist die Behandlung mit expressionsfähiger DNA, die für die relevanten Allergene kodiert (immuntherapeutische DNA-Vakzinierung). Experimentelle Belege für die allergenspezifische Beeinflussung der Immunantwort wurden an Nagern durch Injektion von Allergen-kodierender DNA erbracht (Hsu et al., 1996, Nature Medicine 2 (5): 540-544).

Die der vorliegenden Erfindung zugrunde liegende Aufgabe bestand nun in der Bereitstellung neuartiger Varianten der Gruppe-1-Allergene der *Poaceae* auf Protein- und DNA-Ebene, die sich bei weitgehendem Erhalt der T-Zell-Reaktivität durch eine reduzierte IgE-Aktivität auszeichnen und daher für die kurative und präventive spezifische Immuntherapie sowie die immuntherapeutische DNA-Vakzinierung geeignet sind.

### Abbildungen

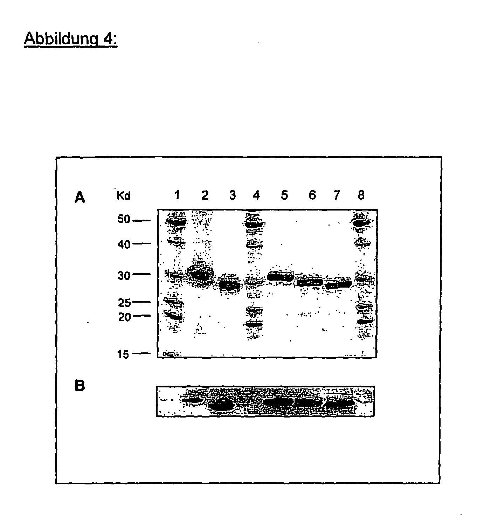

Abbildung 1: Alignment Phl p 1-homologer Aminosäuresequenzen (Sequenzen reifer Proteine deduziert aus cDNA-Sequenzen) von Spezies der Poaceae: Poa pratensis (Poa p), Holcus lanatus (Hol I), Lolium perenne (Lol p), Cynodon dactylon (Cyn d), Oryza sativa (Ory s) und Phalaris aquatica (Pha a), Proteinsequenzen deduziert von cDNA-Sequenzen der "GenBank"-Datenbank des National Center for Biotechnology Information (NCBI, Bethesda, USA), Nummerierung: Aminosäurepositionen reifer Proteine, Durch Unterstreichung gekennzeichnet: Zu Phl p 1-Sequenz differente Aminosäuren, Schwarzer Rahmen: Cysteine

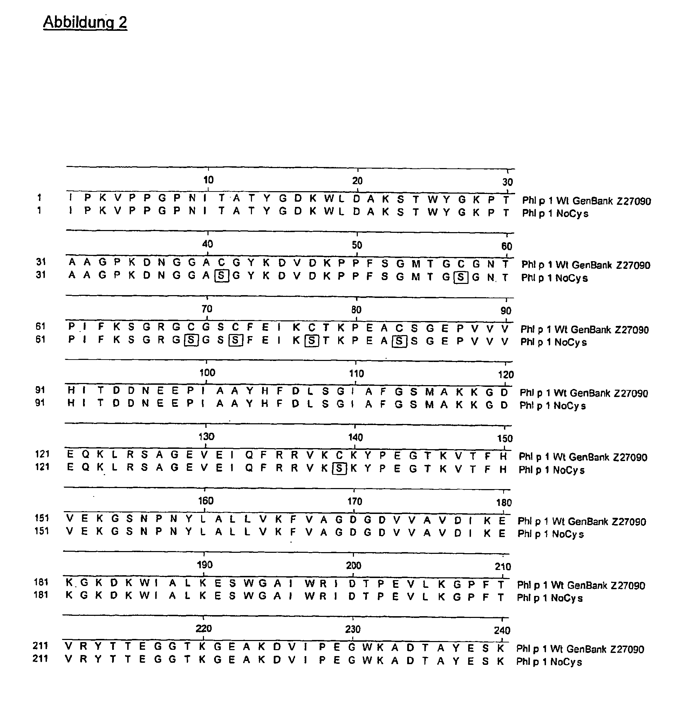

Abbildung 2: Alignment von Aminosäuresequenzen des prozessierten Phl p1 Wildtyp-Proteins und der Variante Phl p1 NoCys, Phl p1 Wt (Wildtyp): Proteinsequenz deduziert aus cDNA-Sequenz ("GenBank"-Datenbankeintrag Z27090 des National Center for Biotechnology Information (NCBI), Bethesda, USA), Nummerierung: Aminosäurepositionen des reifen Proteins, Schwarz eingerahmt: Aminosäuresubstitutionen Cystein gegen Serin in Protein Phl p 1 NoCys

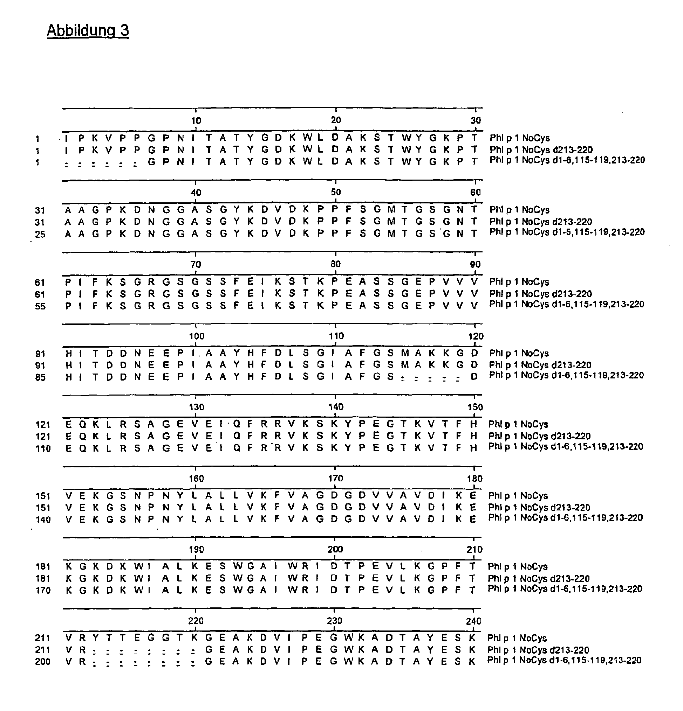

Abbildung 3: Alignment von Aminosäuresequenzen der beispielhaft dargestellten hypoallergenen Varianten Phl p 1 NoCys, Phl p 1 NoCys Δ213-220 und Phl p 1 NoCys Δ1-6, 115-119, 213-220,
   Nummerierung: Aminosäurepositionen, Durch Unterstreichung gekennzeichnet: Deletionen
Abbildung 4: SDS-PAGE und Identitätsüberprüfung der rekombinanten Varianten Phl p 1 NoCys, Phl p 1 NoCys Δ213-220 und Phl p 1 NoCys Δ1-6, 115-119, 213-220
   A: SDS-PAGE
   B: Western Blot mit aPhl p 1-Antikörper (Allergopharma)
   1. Markerproteine
   2. nPhl p 1 *
   3. rPhl p 1 Wt (- His-tag)*
   4. Markerproteine
   5. Phl p 1 NoCys (+ His-tag)
   6. Phl p 1 NoCys D213-220 (+ His-tag)
   7. Phl p 1 NoCys D1-6, 115-119, 213-220 (+ His-tag)
   8. Markerproteine
   * Proben reduziert (Dithiotreitol)
Abbildung 5: Streifentest zur Überprüfung der IgE-Bindungsfähigkeit von Phl p 1 NoCys, Phl p 1 NoCys Δ213-220 und Phl p 1 NoCys Δ1-6, 115-119, 213-220 unter nicht-denaturierenden Bedingungen
   1) rPhl p 1 Wt
   2) Phl p 1 NoCys
   3) Phl p 1 NoCys D213-220
   4) Phl p 1 NoCys D1-6, 115-119, 213-220
   5) rPhl p 1 Wt
   TP: Gesamtproteinfärbung
   P: Seren klinisch definierter Gräserpollen-Allergiker
Abbildung 6: Nachweis der reduzierten IgE-Reaktivität von Phl p 1 NoCys, Phl p 1 NoCys Δ213-220 und Phl p 1 NoCys Δ1-6, 115-119, 213-220 mittels EAST-Inhibitionstest mit vier repräsentativen Seren von Gräserpollen-Allergikern (P)
Abbildung 7: Nachweis der Hypoallergenität von Phl p 1 NoCys mittels Basophilen-Aktivierungstest mit Basophilen von vier Gräserpollen-Allergikern (P)
Abbildung 8: Nachweis der Hypoallergenität von Phl p 1 NoCys Δ213-220 mittels Basophilen-Aktivierungstest mit Basophilen von vier Gräserpollen-Allergikern (P)
Abbildung 9: Nachweis der Hypoallergenität von Phl p 1 NoCys Δ1-6, 115-119, 213-220 mittels Basophilen-Aktivierungstest mit Basophilen von vier Gräserpollen-Allergikern (P)

Die Arbeiten, die zu den aufgefundenen Varianten führten, wurden anhand von Phl p 1 als Modellallergen durchgeführt. Aus den in Abb. 1 gezeigten Sequenz-Alignments wird deutlich, daß aufgrund der hohen Homologie innerhalb der Gruppe 1 dieselben Ergebnisse erhalten worden wären, wenn von einem anderen Gruppe 1-Allergen ausgegangen worden wäre.
So ist auch davon auszugehen, daß die vor- und nachstehend aufgeführten Ergebnisse auch auf Sec c 1 aus *Secale cereale* übertragbar sind bzw. unter Verwendung von Sec c 1 erhalten worden wären, auch wenn für dieses Gruppe 1-Allergen die Sequenz noch unbekannt ist.

Gegenstand der vorliegenden Erfindung sind somit Varianten der Gruppe-1-Allergene der *Poaceae*, welche durch eine gegenüber den bekannten Wildtyp-Allergenen verringerte IgE-Reaktivität sowie eine erhaltene Reaktivität mit T-Lymphozyten gekennzeichnet sind. Vorzugsweise sind diese Gruppe-1- Allergene Phl p 1, Poa p 1, Hol p 1, Lol p 1, Cyn d 1, Ory s 1 und Pha a 1 aus *Phleum pratense, Lolium perenne, Poa pratensis, Holcus lanatus, Cynodon dactylon, Oryza sativa* und *Phalaris aquatica.* Stärker bevorzugt sind Phl p 1, Poa p 1, Hol p 1, Lol p 1 bzw. Pha a 1 und ganz besonders bevorzugt Phl p 1.

Ausgangspunkt für die Konstruktion der hypoallergenen Varianten der Gruppe 1-Allergene ist die cDNA des Phl p 1 Wildtyps, die mit Hilfe spezifischer Primer durch Polymerase-Kettenreaktion (PCR) aus der GesamtcDNA von Pollen von *Phleum pratense* isoliert wurde ("GenBank" Eintrag Z27090; NCBI, Bethesda, USA) (SEQ ID NO 1).
Von der cDNA-Sequenz des Phl p 1 Wildtyps wurde die Aminosäuresequenz gemäß SEQ ID NO 2 deduziert.
Das aus 240 Aminosäuren bestehende, in der natürlichen Form glykosylierte Phl p 1 ist - wie alle Gruppe 1-Allergene (siehe Abb. 1) - durch die Existenz von sieben Cysteinen des reifen Moleküls gekennzeichnet. Mit der Ausnahme von Cyn d 1 und Ory s 1 haben diese Aminosäurepositionen bei allen Gruppe 1-Allergenen die Nummern 41, 57, 69, 72, 77, 83 und 139 (Petersen et al., 1995, J. Allergy Clin. Immunol 95: 987-994).

Phl p 1 konnte in *E. coli* als nicht-glykosyliertes Protein exprimiert werden. Das rekombinante Wildtyp-Protein (rPhl p 1 wt) reagierte mit IgE-Antikörpern von Gräserpollenallergikern, die eine Reaktivität mit natürlichem gereinigten Phl p 1 (nPhl p 1) aufwiesen (Petersen et al., 1998, Clin. Exp. Allergy 28: 315-321).

### Detaillierte Beschreibung der Erfindung

### Herstellung und Charakterisierung hypoallergener Phl p 1-Varianten

Ausgehend von der beschriebenen cDNA von rPhl p 1 wt wurden gentechnisch veränderte rekombinante Varianten von Phl p 1 hergestellt.

Die Aminosäuresequenz der rekombinanten Variante Phl p 1 NoCys (SEQ ID NO 4) weist anstelle der im Wildtyp vorkommenden sieben Cysteine sieben Serinreste auf (Abb. 2). Die Variante Phl p 1 NoCys diente als Ausgangspunkt für die Konstruktion von unterschiedlichen Deletionsmutanten. Bei diesen sind jeweils einzelne Abschnitte von 15 bis 90 bp Länge oder Kombinationen dieser Abschnitte der cDNA kodierend für Phl p 1 NoCys deletiert, wodurch entsprechende Deletionen der Aminosäuren 1-6, 1-30, 92-104, 115-119, 175-185 und 213-220 in den Polypeptidketten der in *E. coli* exprimierten Proteine resultieren:
Phl p 1 NoCys Δ1-6 (SEQ ID NO 5 und 6), Phl p 1 NoCys Δ1-30 (SEQ ID NO 7 und 8), Phl p 1 NoCys Δ92-104 (SEQ ID NO 9 und 10), Phl p 1 NoCys Δ115-119 (SEQ ID NO 11 und 12), Phl p 1 NoCys Δ175-185 (SEQ ID NO 13 und 14), Phl p 1 NoCys Δ213-220 (SEQ ID NO 15 und 16), sowie Phl p 1 NoCys Δ1-6, 115-119, 213-220 (SEQ ID NO 17 und 18).
   Die Expression der rekombinanten Proteine erfolgte als Histidin-Fusionsproteine in *Escherichia coli.* Die immunologische Charakterisierung wurde mit solchen Fustionspoteinen durchgeführt.

Zunächst wurden die rekombinanten Varianten nach Immobilisierung an eine Nitrocellulose-Membran auf die Fähigkeit, von IgE-Antikörpern eines repräsentativen Serumpools und von IgE-Antikörpern von individuellen Seren von Gräserpollenallergikern gebunden zu werden, untersucht (Streifentest). Bei diesem Verfahren wurde überraschenderweise eine verminderte Bindung von 1gE-Antikörpern an Phl p 1 NoCys beobachtet. Dieses Ergebnis wurde durch einen IgE-Inhibitionstest (EAST), bei dem die IgE-Bindungsfähigkeit eines nicht-immobilisierten Proteins an IgE-Antikörper in Lösung untersucht wird, bestätigt.

Gegenstand der vorliegenden Erfindung sind daher insbesondere solche Gruppe 1-Allergenvarianten, bei denen die Cysteine der dem reifen Phl p 1 Protein entsprechenden Aminosäurepositionen 41, 57, 69, 72, 77, 83 und 139 entfernt oder gegen eine andere Aminosäure ersetzt werden. Besonders bevorzugt sind dabei entsprechende Varianten von Phl p 1, Poa p 1, Hol p 1, Lol p 1 oder Pha a 1, insbesondere von Phl p 1.

Eine verminderte Bindung von IgE-Antikörpern wird bereits dann erhalten, wenn mindestens zwei der 7 Cysteine ersatzlos entfernt oder gegen eine andere Aminosäure ersetzt werden. Vorzugsweise werden jedoch alle 7 Cysteine gegen Serin ausgetauscht.

Mittels eines Aktivierungstests von basophilen Granulozyten von Gräserpollenallergikern wurden die Auswirkungen der reduzierten IgE-Bindungsfähigkeit von Phl p 1 NoCys auf die funktionelle Wirkung bei der Vernetzung von membrangebundenem IgE der Effektorzellen und deren Aktivierung *in vitro* untersucht. Phl p 1 NoCys zeigte hier eine deutlich geringere Aktivierung von basophilen Granulozyten im Vergleich zu rPhl p 1 wt und somit eine funktionell reduzierte Allergenität.

Die auf Basis von Phl p 1 NoCys hergestellten unterschiedlichen Deletionsmutanten wurden nach dem gleichen Verfahren hinsichtlich der IgE-Bindungsfähigkeit (Streifentest, EAST) und der funktionellen Wirkung (Basophilenaktivierung) untersucht. Überraschenderweise zeigten die Deletionsmutanten besonders starke hypoallergene Eigenschaften.

Gegenstand der vorliegenden Erfindung sind daher weiterhin solche Gruppe 1-Allergenvarianten, bei denen - wahlweise zusätzlich zu den zuvor beschriebenen Varianten mit entferntem bzw. ersetztem Cys - mindestens ein Bereich oder eine Kombination von Bereichen, die den Aminosäuren 1-6, 1-30, 92-104, 115-119, 175-185 und 213-220 der Primärsequenz des reifen Phl p 1 Proteins entsprechen, gegenüber dem Wildtyp-Allergen fehlen.
Besonders bevorzugt sind dabei die entsprechenden Deletionsmutanten der Gruppe 1-Allergene Phl p 1, Poa p 1, Hol p 1, Lol p 1 und Pha a 1. Ganz besonders bevorzugt sind die entsprechenden Phl p1-Varianten.

Insbesondere bevorzugt und daher ebenfalls Erfindungsgegenstand sind diejenigen Gruppe 1-Allergenvarianten, bei denen ausschließlich die Aminosäuren 213-220 bzw. gleichzeitig die Aminosäuren 1-6 und 115-119 entsprechend der reifen Phl p 1-Sequenz fehlen. Stärker bevorzugt sind hierbei wieder die Allergene Phl p 1, Poa p 1, Hol p 1, Lol p 1 und Pha a 1, wobei Phl p 1 ganz besonders bevorzugt ist.

Die der Wirksamkeit der spezifischen Immuntherapie zugrundeliegende T-Zell-Reaktivität der hypoallergenen Phl p1-Varianten wurde durch einen Proliferationstest mit Phl p 1 -spezifischen T-Lymphozyten von Gräserpollenallergikern *in vitro* überprüft. Die modifizierten Allergene zeigten eine weitgehend erhaltene T-Zell-Reaktivität, was eine immuntherapeutische Verwendung der hypoallergenen Phl p1-Varianten ermöglicht.

Es bietet sich an, die erfindungsgemäßen Allergenvarianten, ausgehend von der klonierten DNA-Sequenz mit Hilfe gentechnischer Methoden herzustellen. Grundsätzlich kann es sich aber auch um chemische Modifikationen des nativen Allergenextrakts handeln (Fiebig, 1995, Allergo J. 4 (7), 377-382).
Naturgemäß sind über die in der vorliegenden Patentanmeldung beschriebenen Variationen von Gruppe-1-Allergenen auch weitere Veränderungen an anderen Positionen - etwa zur Erhöhung der Hypoallergenität - möglich. Bei diesen Modifikationen kann es sich beispielsweise um Aminosäure-Insertionen, -Deletionen und -Austausche, Aufspaltungen des Proteins in Fragmente sowie Fusionen des Proteins oder seiner Fragmente mit anderen Proteinen oder Peptiden handeln.
Gegenstand ist weiterhin ein DNA-Molekül, kodierend für eine zuvor beschriebene Allergenvariante, ein rekombinanter Expressionsvektor, enthaltend dieses DNA-Molekül sowie ein Wirtsorganismus, transformiert mit besagtem DNA-Molekül oder besagtem Expressionsvektor. Geeignete Wirtsorganismen können pro- oder eukaryontische, ein- oder mehrzellige Organismen wie Bakterien oder Hefen sein. Ein erfindungsgemäß bevorzugter Wirtsorganismus ist *E*. *coli.*

Weiterhin betrifft die Erfindung ein Verfahren zur Herstellung einer erfindungsgemäßen Allergenvariante durch Kultivieren des besagten Wirtsorganismus und Gewinnung der entsprechenden Allergenvariante aus der Kultur.

Gegenstand der vorliegenden Erfindung sind außerdem die zuvor beschriebenen Allergenvarianten, DNA-Moleküle und Expressionsvektoren in ihrer Eigenschaft als Arzneimittel.

Weiterhin sind Gegenstand der vorliegenden Erfindung pharmazeutische Zubereitungen enthaltend mindestens eine dieser Allergenvarianten bzw. ein entsprechendes DNA-Molekül oder einen entsprechenden Expressionsvektor sowie gegebenenfalls weitere Wirk- und/oder Hilfsstoffe zur Behandlung von Allergien, an deren Auslösung Gruppe-1-Allergene der Poaceae beteiligt sind, bzw. zur immuntherapeutischen Vakzinierung von Patienten mit Allergien, an deren Auslösung Gruppe-1-Allergene der Poaceae beteiligt sind und/oder zur Prävention solcher Allergien.
Handelt es sich um pharmazeutische Zubereitungen des zweiten Typs (enthaltend mindestens ein DNA-Molekül oder einen Expressionsvektor), so enthalten diese Zubereitungen vorzugsweise weiterhin Aluminiumhydroxid, ein immunstimulatorisches CpG-haltiges Oligonukleotid oder eine Kombination beider als Hilfsstoffe.

Pharmazeutische Zubereitungen im Sinne dieser Erfindung können als Therapeutika in der Human- oder Veterinärmedizin verwendet werden. Als Trägerstoffe kommen organische oder anorganische Substanzen in Frage, die sich für die parenterale Applikation eignen und mit erfindungsgemäßen Gruppe-1-Allergenvarianten nicht reagieren. Zur parenteralen Anwendung dienen insbesondere Lösungen, vorzugsweise ölige oder wässrige Lösungen, ferner Suspensionen, Emulsionen oder Implantate. Die erfindungsgemäßen Allergenvarianten können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten. Weiterhin können durch entsprechende Formulierung der erfindungsgemäßen Allergenvarianten Depotpräparate, beispielsweise durch Adsorption an Aluminiumhydroxid, erhalten werden.

Schließlich ist Gegenstand der vorliegenden Erfindung die Verwendung mindestens einer erfindungsgemäßen Allergenvariante bzw. eines erfindungsgemäßen DNA-Moleküls oder eines erfindungsgemäßen Expressionsvektors zur Herstellung eines Arzneimittels zur Behandlung von Allergien, an deren Auslösung Gruppe-1-Allergene der *Poaceae* beteiligt sind bzw. zur immuntherapeutischen Vakzinierung von Patienten mit Allergien, an deren Auslösung Gruppe-1-Allergene der *Poaceae* beteiligt sind und/oder zur Prävention solcher Allergien.

Die Herstellung der Varianten Phl p 1 NoCys, Phl p 1 NoCys Δ213-220 und Phl p 1 NoCys Δ1-6, 115-119, 213-220 (Abb. 3) sowie deren immunologische Charakterisierung ist im folgenden beispielhaft für die hypoallergenen Phl p 1 -Varianten mit den oben beschriebenen gentechnischen Modifikationen dargestellt.

### Expression und Reinigung rekombinanter Phl p 1-Varianten

Die Expression der rekombinanten Proteine erfolgte als Histidin-Fusionsproteine (Expressionsvektor pProExHT; Invitrogen, Carlsbad, USA) in *Escherichia coli* (Stamm JM109). rPhl p 1 wt sowie die Varianten wurden zunächst durch die spezifische Bindung der N-terminalen Histidinreste an eine Ni²⁺-Chelat-Matrix (Immobilized-Metal-lon-Affinity-Chromatography, IMAC) und nachfolgend durch präparative Gelfiltration (Size-Exclusion-Chromatography, SEC) gereinigt. Die Reinheit der eluierten Proteine wurde durch SDS-PAGE und analytische SEC kontrolliert (Abb. 4a). Die Identität der gereinigten Proteine wurde durch die Bindung eines Phl p 1-spezifischen monoklonalen Antikörpers nachgewiesen (Abb. 4b).

### Nachweis der reduzierten IgE-Bindung der rekombinanten Phl p 1-Varianten

Eine einfache Testmethode zur Bestimmung der IgE-Reaktivität von spezifischem IgE aus Allergikerseren an membrangebundene Testproteine ist der Streifentest.
Dafür werden die Testsubstanzen in gleicher Konzentration und Menge nebeneinander an einen Streifen von Nitrocellulose-Membran unter nicht-denaturierenden Bedingungen gebunden. Eine Reihe solcher Membranstreifen kann parallel mit unterschiedlichen Allergikerseren inkubiert werden. Nach einem Waschschritt werden die spezifisch gebundenen IgE-Antikörper durch eine Farbreaktion, vermittelt von einem Anti-human IgE/ Alkalische Phosphatase-Konjugat, auf der Membran sichtbar gemacht.

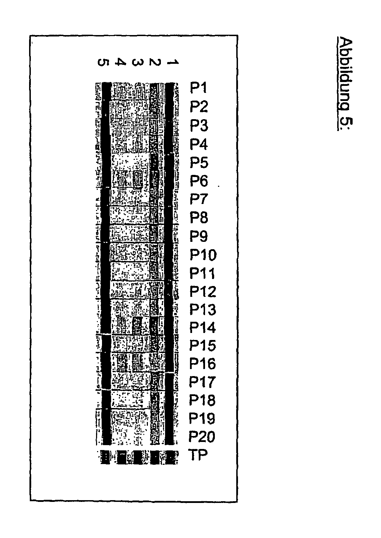

Beispielhaft für die beschriebenen modifizierten Phl p 1-Moleküle werden hier die Ergebnisse des Streifentests bezüglich Phl p 1 NoCys, Phl p 1 NoCys Δ213-220 und Phl p 1 NoCys Δ1-6, 115-119, 213-220 unter Einsatz von individuellen Gräserpollen-Allergikerseren dargestellt (Abb. 5). Es kamen nur Seren von Allergikern mit einem starken IgE-Titer gegen natürliches Phl p 1 zur Anwendung. Die IgE-Antikörper dieser Patienten reagieren ebenso mit dem rekombinanten Äquivalent rPhl p 1 wt.

Es wird deutlich, dass die Phl p 1-spezifischen IgE-Antikörper aller Patientenseren die rekombinante Variante Phl p 1 NoCys vermindert binden, nicht aber das Wildtypallergen Phl p 1.

Eine noch stärkere Reduktion der IgE-Bindungsfähigkeit wird durch das zusätzliche Entfernen bestimmter Sequenzabschnitte erzielt, was anhand an der Variante Phl p 1 NoCys Δ213-220 dargestellt ist. Die Variante Phl p 1 NoCys Δ213-220 zeigt mit allen überprüften Allergikerseren eine sehr stark verminderte lgE-Bindungsfähigkeit gegenüber dem nichtveränderten rekombinanten Wildtyp-Protein. Eine weitere Reduktion der IgE-Bindungsfähigkeit von Phl p 1 an IgE-Antikörper bestimmter Seren kann durch Kombination mehrerer Deletionen erzielt werden, was an dem Testergebnis der Variante Phl p 1 NoCys Δ1-6, 115-119, 213-220 mit dem Gräserpollen-Allergikerserum P14 erkennbar ist (Abb. 5).
Somit wird deutlich, dass sowohl die Substitution von Cysteinen als auch die Deletion von spezifischen Sequenzabschnitten die lgE-Bindungsfähigkeit des Phl p 1-Moleküls herabsetzt.

Im Gegensatz zu dem Streifentest können mit dem EAST-Hemmtest (Enzyme Allergosorbent Test) Allergen/ IgE-Interaktionen in Lösung untersucht werden, womit eine störende Maskierung von Epitopen der Testsubstanz durch die Immobilisierung an die Membran grundsätzlich ausgeschlossen werden kann.
Der EAST-Hemmtest wird wie folgt ausgeführt. Mikrotiterplatten werden mit Allergenen, hier nPhl p 1, beschichtet. Nach Entfernung der nicht gebundenen Allergenmoleküle durch Waschen wird die Platte zur Vermeidung späterer unspezifischer Bindungen mit Rinderserumalbumin blockiert. IgE-Antikörper von Allergikern, als repräsentativer Pool von Einzelseren (Serum-Pool) oder als Einzelserum, wird in geeigneter Verdünnung mit den Allergen-beschichteten Mikrotiterplatten inkubiert. Die Menge der Allergengebundenen IgE-Antikörper wird über ein Anti-hlgE/ Alkalische Phosphatase-Konjugat durch die Umsetzung eines Substrates zu einem farbigen Endprodukt photometrisch quantifiziert.

Die Bindung der IgE-Antikörper wird durch ein lösliches Allergen bzw. die zu prüfende Substanz (rekombinantes modifiziertes Allergen) in Abhängigkeit von der Konzentration substanzspezifisch gehemmt.

Beispielhaft für die beschriebenen modifizierten Phl p 1-Moleküle werden hier die Testergebnisse bezüglich Phl p 1 NoCys, Phl p 1 NoCys Δ213-220 und Phl p 1 NoCys Δ1-6, 115-119, 213-220 im Vergleich zu dem Referenzmolekül nPhl p 1 dargestellt.

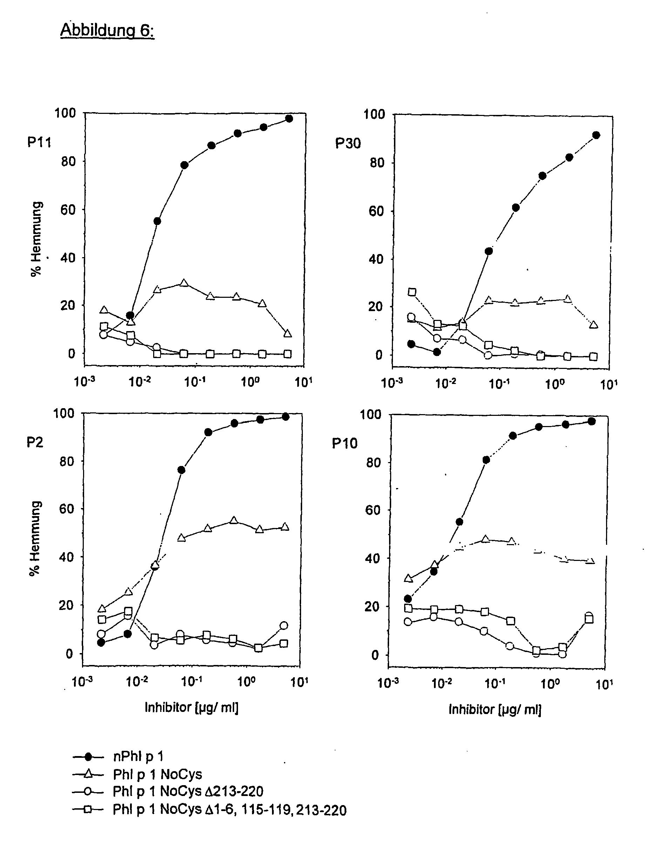

Die in Abbildung 6 dargestellten repräsentativen IgE-Inhibitionstests mit vier Individualseren von Gräserpollen-Allergikern zeigen, dass selbst mit hohen Konzentrationen der Variante Phl p 1 NoCys (bis 5 µg/ ml) nur ca. 20-50% der maximalen Hemmwirkung des unveränderten natürlichen Allergens nPhl p1 erzielt wurde. Die niedrigere maximale Hemmwirkung deutet auf einen Verlust von IgE-Epitopen hin.
Die Kurvenverläufe der Varianten Phl p 1 NoCys Δ213-220 und Phl p 1 NoCys Δ1-6, 115-119, 213-220 zeigen eine noch geringere IgE-Bindungsfähigkeit dieser Phl p 1-Varianten auf. Eine Hemmwirkung konnte individuell variierend nicht oder nur in sehr geringer Stärke (0-20% der maximalen Hemmwirkung) nachgewiesen werden.
Übereinstimmend mit dem Ergebnis des Streifentests kann somit belegt werden, dass durch Einfügen zusätzlicher gezielter Deletionen die lgE-Bindungsfähigkeit von Phl p 1 weiter reduziert wird.

### Nachweis der Hypoallergenität der rekombinanten Phl p 1-Varianten durch Basophilen-Aktivierungstest

Die funktionelle Reduktion der Allergenität wurde mittels eines Basophilenaktivierungstests *in vitro* bestimmt. Für den Basophilenaktivierungstest wird heparinisiertes Vollblut von Gräserpollen-Allergikern mit verschiedenen Konzentrationen der Testsubstanzen inkubiert. Die allergenen Substanzen werden von den FcεRI-gebundenen IgE-Antikörpern der Basophilen spezifisch gebunden und führen zu einer Vernetzung der FcεRI-Moleküle.
Diese Allergen-induzierte lge-vermittelte FcεRI-Vernetzung führt zur Aktivierung der Basophilen. Die Aktivierung ist der erste Schritt in der allergischen Reaktion dieser Effektorzellen. Die anschließende Signaltransduktion resultiert in der Degranulation der Effektorzellen und somit dem Auslösen der allergischen Reaktionen *in vivo.*

*In vitro* kann die Allergen-induzierte Aktivierung von basophilen Granulozyten durch Quantifizierung der Expression eines mit der Signaltransduktion der IgE-Rezeptor-Vernetzung gekoppelten Oberflächenproteins (CD203c) nachgewiesen werden (Kahlert et al., 2003, Cli., Exp. Allergy 33: 1266-72). Die Zahl der exprimierten CD203c-Proteine auf einer Zelle und der Prozentwert der aktivierten Zellen eines Zellpools wird über die Bindung eines fluoreszenzmarkierten monoklonalen Antikörpers an den Oberflächenmarker und anschließende Analyse durch fluoreszenzaktivierte Durchflusszytometrie hochsensitiv gemessen.
Als Referenzsubstanzen wurde hier das gereinigte natürliche Phl p 1 (nPhl p 1) parallel zu den Testsubstanzen eingesetzt. Beispielhaft für die beschriebenen modifizierten Phl p 1-Moleküle werden hier die Ergebnisse Phl p 1 NoCys, Phl p 1 NoCys Δ213-220 und Phl p 1 NoCys Δ1-6, 115-119, 213-220 dargestellt.

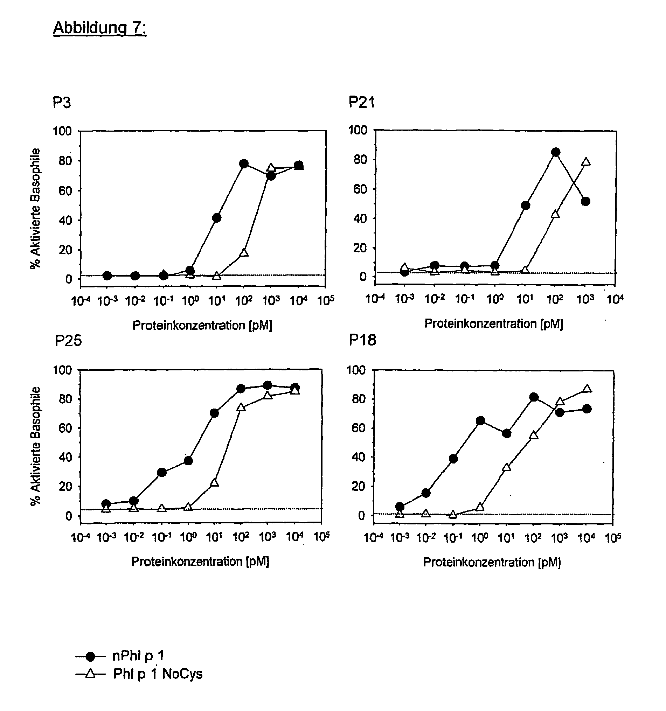

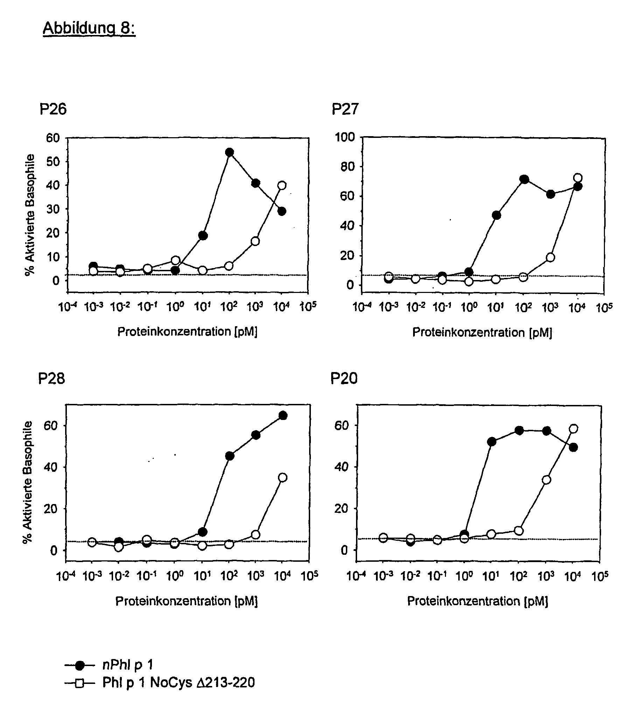

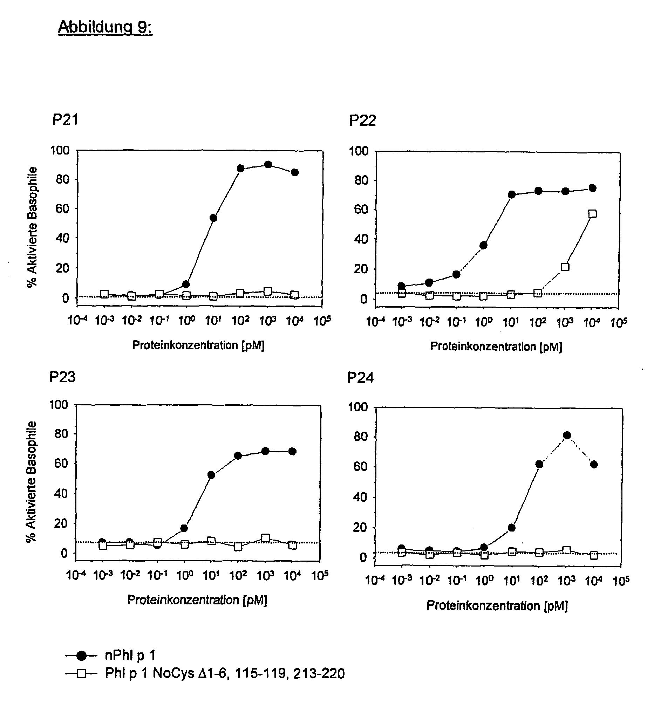

Repräsentative Testergebnisse der Variante Phl p 1 NoCys mit Basophilen von vier klinisch definierten Allergikern sind in Abbildung 7 als Kurvenverläufe gezeigt. Die Reduktion der allergenen Wirksamkeit der Variante Phl p 1 NoCys relativ zu nPhl p 1 Wildtyp wird durch die Verschiebung der Aktivierungskurven deutlich.
Die Testergebnisse der Varianten Phl p 1 NoCys Δ213-220 und Phl p 1 NoCys Δ1-6, 115-119, 213-220 weisen im Einklang mit den Ergebnissen des Streifentests und des IgE-Inhibitionstests auf eine noch stärkere Reduktion der relativen allergenen Wirksamkeit hin, wie in Abbildung 8 und 9 anhand repräsentativer Kurvenläufe gezeigt wird.
Während in einem Konzentrationsbereich der Testsubstanzen von 100-1000 pM durch das natürliche Allergen bereits ein maximaler Anteil von Basophilen aktiviert wurde, konnte keine oder eine nur sehr geringe Basophilen-Aktivierung bei Einsatz der modifizierten Allergene Phl p 1 NoCys Δ213-220 und Phl p 1 NoCys Δ1-6, 115-119, 213-220 nachgewiesen werden.
Die allergene Wirksamkeit der Variante Phl p 1 NoCys Δ213-220 war, wie über die A50-Werte der Kurvenverläufe berechnet werden kann, ~ 100-1000 fach und die der Variante Phl p 1 NoCys Δ1-6, 115-119, 213-220 mehr als 1000 fach gegenüber der Referenz nPhl p 1 erniedrigt (A50: Allergenkonzentration bei 50% der Zahl maximal aktivierter Basophilen).

### T-Zell-Reaktivität der hypoallergenen Phl p 1-Varianten

T-Helfer-Lymphozyten reagieren mit Peptidfragmenten der Allergene (ca. 12-25 Aminosäuren), die durch enzymatischen Abbau in Antigenpräsentierenden Zellen (APZ) entstehen und nach Einlagerung der geeigneten Peptide in die individuellen MHC-Klasse II-Moleküle an der Oberfläche der APZ den T-Zellen präsentiert werden. Diese allergenspezifische Aktivierung der T-Helfer-Lymphozyten ist die Voraussetzung für Proliferation und für die funktionelle Differenzierung (TH1 und TH2). Die Beeinflussung der allergenspezifischen T-Lymphozyten durch die Behandlung mit Allergen oder einem Allergenderivat bei der Hyposensibilisierung wird als Schlüssel für die therapeutische Wirksamkeit angesehen.

Zur Untersuchung der T-Zell-Reaktivität werden oligoklonale T-Zell-Linien von Graspollen-Allergikem unter Stimulation mit nPhl p 1 - oder rPhl p 1 wt -Molekülen nach üblichen Verfahren etabliert. In einem Proliferationstest wurden die unterschiedlichen T-Zell-Linien mit den Referenz-Allergenen nPhl p 1 und rPhl p 1 wt sowie den modifizierten rekombinanten Phl p 1-Varianten stimuliert. Die Proliferationsrate wurde durch den Einbau von [³H]-Thymidin mit den üblichen Verfahren bestimmt.

Beispielhaft für die beschriebenen modifizierten Phl p 1-Moleküle sind hier die Ergebnisse des Proliferationstest von Phl p 1 NoCys, Phl p 1 NoCys Δ213-220 und Phl p 1 NoCys Δ1-6, 115-119, 213-220 dargestellt.

Die in Tabelle 1 dargestellten Ergebnisse mit T-Zell-Linien von acht Gräserpollen-Allergikern zeigen, dass die T-Lymphozyten durch die rekombinanten Allergenvarianten zur Proliferation stimuliert werden konnten. Die T-Zellreaktivität von Phl p 1 NoCys, Phl p 1 NoCys Δ213-220 und Phl p 1 NoCys Δ1-6, 115-119, 213-220 ist im Vergleich zu den unveränderten natürlichen und rekombinanten Wildtyp-Allergenen nur wenig erniedrigt, was die Erhaltung der entscheidenden T-Zell-Epitope nachweist.

**Tabelle 1: Nachweis der T-Zellreaktivität von Phl p 1 NoCys, Phl p 1 NoCys Δ213-220 und Phl p 1 NoCys Δ1-6, 115-119, 213-220 mittels Proliferationstests mit Phl p 1-reaktiven T-Zell-Linien (TCL)**

| | | Stimulationsindex¹ | | | | |
|---|---|---|---|---|---|---|
| Donor² | TCL | nPhl p 1 | rPhl p 1 Wt | Phl p 1 NoCys | Phl p 1 NoCys Δ213-220 | Phl p 1 NoCys Δ1-6, 115-119, 213-220 |
| A | 11.16 | 3,2 | 4,7 | 3,7 | 3,5 | 3,9 |
| B | 21.1 | 23,5 | 13,9 | 12,1 | 13,0 | 12,3 |
| C | 60.51 | 3,9 | 5,7 | 3,9 | 4,2 | 2,0 |
| D | 104.8 | 2,6 | 4,6 | 4,1 | 3,4 | 3,0 |
| E | 10.27 | 26,4 | 27,8 | 32,7 | 30,8 | 31,4 |
| F | 41.8 | 2,7 | 4,9 | 3,5 | 3,0 | 3,1 |
| G | 55.74 | 7,6 | 8,4 | 4,9 | 4,6 | 4,5 |
| H | 57.43 | 3,1 | 4,0 | 3,0 | 2,6 | 2,9 |

| | | | | | | |
|---|---|---|---|---|---|---|
| ¹ Berechnet von [³H]-Messwerten. cpm-Messwerte Allergen-stimulierter Zellkulturen/ cpm-Messwerte unstimulierter Zellkulturen ²Donor: Klinisch definierte Gräserpollen-Allergiker *Gentechnische Konstruktion von hypoallergenen Phl p 1-Varianten* | | | | | | |

### Beispiel 1: Phl p 1 NoCys

Zur Konstruktion der Variante Phl p 1 NoCys (SEQ ID NO 3 und 4) wurden sechs PCR-Schritte ausgehend von der cDNA von rPhl p 1 wt ("GenBank" Eintrag Z27090; NCBI, Bethesda, USA) durchgeführt. Die Punktmutationen wurden eingeführt, indem spezifische PCR-Primer eingesetzt wurden, die anstelle von für Cystein kodierenden Kodons solche für Serin enthielten (Primersequenzen s. Tab. 2).

Schritt 1 - Herstellung des N-terminalen DNA-Fragments "Phl p 1 [C41 S, C57S, C69S] (bp 1-212)": Durch Amplifizierung langer überlappender Oligonukleotide (P 1-63, P 49-111, P 97-158 und P 144-212) mittels PCR wurde ein DNA-Fragment mit den Mutationen C41 S, C57S, C69S generiert.

Schritt 2 - Herstellung des C-terminalen DNA-Fragments "Phl p 1 [C69S, C72S, C77S, C83S] (bp 193-720)": PCR der Phl p 1 wt-cDNA mit den Primern P 193-261 und P 703-720 *Hind*III*.*

Schritt 3 - Herstellung der DNA kodierend für "Phl p 1 [C41 S, C57S, C69S, C72S, C77S, C83S] (bp 1-720)": PCR der überlappenden Fragmente "Phl p 1 [C41S, C57S, C69S] (bp 1-212)" und "Phl p 1 [C69S, C72S, C77S, C83S] (bp 193-720)" mit den Primern P 1-63 und P 703-720 *Hind*III.

Schritt 4 - Herstellung des N-terminalen DNA-Fragments "Phl p 1 [C41 S, C57S, C69S, C72S, C77S, C83S, C139S] (bp 1-428)": PCR der cDNA von "Phl p 1 [C41S, C57S, C69S, C72S, C77S, C83S] (bp 1-720)" mit den Primern P 1-63 und P 406-428 as.

Schritt 5 - Herstellung des C-terminalen DNA-Fragments "Phl p 1 [C139S] (bp 406-720)": PCR der cDNA von rPhl p 1 wt mit den Primern P 406-428s und P 703-720 *Hind*III*.*

Schritt 6 - Herstellung der vollständigen DNA kodierend für Phl p 1 NoCys: PCR der überlappenden Fragmente "Phl p 1 [C41 S, C57S, C69S, C72S, C77S, C83S, C139S] (bp 1-428)" und "Phl p 1 [C139S] (bp 406-720) mit den Primern P 1-63 und P 703-720 *Hind*III*".*

Die für Phl p 1 NoCys kodierende DNA wurden mit dem Restriktionsenzym *Hind*III verdaut und über die Restriktionsschnittstellen Ehel und *Hind*III in den Expressionsvektor pProExHT (Invitrogen, Carlsbad, USA) ligiert, und anschließend vollständig sequenziert.

**Tabelle 2: Aufstellung der zur Herstellung von Phl p 1 NoCys, Phl p 1 NoCys Δ213-220 und Phl p 1 NoCys Δ1-6, 115-119, 213-220 eingesetzten PCR-Primer**

| Primer | Richtung | SEQ ID NO | Sequenz (5'→3') |
|---|---|---|---|
| P 1-18' | sense | 19 | atc ccg aag gtc ccg ccg |
| P 1-63 | sense | 20 | atc ccg aag gtc ccg ccg ggc ccg aac atc acg gcg acc tac ggc gac aag tgg ctg gac gcg |
| P 49-111 | antisense | 21 | gtt gtc ctt ggg acc ggc ggc cgt cgg ctt gcc gta cca ggt gct ctt cgc gtc cag cca ctt |
| P 97-158 | sense | 22 | ggt ccc aag gac aac ggc ggc gcg agc ggg tac aag gac gtg gac aag ccc ccg ttc agc gg |
| P 144-212 | antisense | 23 | gag ccg ct gcc ccg gcc gga ctt gaa gat ggg ggt gtt gcc gga gcc ggt cat gcc gct gaa cgg ggg c |
| P 193-261 | sense | 24 | tcc ggc cgg ggc agc ggc tcc tcc ttc gag atc aag agc acc aag ccc gag gcc tcc tcc ggc gag ccc |
| P 703-720 *Hind*III | antisense | 25 | ggt aag ctt tca ctt gga ctc gta ggc ggt |
| P 406-428 as | antisense | 26 | tcc ggg tac ttg gac ttg acg cg |
| P 406-428 s | sense | 27 | cgc gtc aag tcc aag tac ccg ga |
| P 22-63 (Δ1-18) | sense | 28 | ccg aac atc acg gcg acc tac ggc gac aag tgg ctg gac gcg |
| P 250-318 | antisense | 29 | gtc gaa gtg gta cgc ggc gat ggg ctc ctc gtt gtc gtc ggt gat gtg gac cac cac ggg ctc gcc gga |
| P 301-384 (Δ343-357) | sense | 30 | gcc gcg tac cac ttc gac ctc tcc ggc atc gcg ttc ggg tcc gac gag cag aag ctg cgc agc gcc ggc |
| P 613-720 (Δ637-660)*Hind*III | antisense | 31 | ggt aag ctt tca ctt gga ctc gta ggc ggt gtc ggc ctt cca gcc ctc ggg gat gac gtc ctt ggc ctc gcc gcg gac ggt gaa ggg gcc ctt gag |

| | | | |
|---|---|---|---|
| ¹Zahlenangaben: Positionen der Primer bezogen auf die Nukleotid-Sequenz des Phl p 1 Wildtyp-Proteins (ohne Signalpeptid; "GenBank" Eintrag Z27090; NCBI, Bethesda, USA). Primersequenzen teilweise kodonoptimiert für *E*. *coli.* | | | |

### Beispiel 2: Phl p 1 NoCys Δ213-220

Die DNA-Sequenz kodierend für die Deletionsvariante Phl p 1 NoCys Δ213-220 (SEQ ID NO 15 und 16) wurde mittels PCR der DNA von Phl p 1 NoCys unter Einsatz des 5'-Primers P 1-18 sowie des spezifisch um den zu deletierenden Sequenzbereich verkürzten 3'-Primers P 613-720 (Δ637-660) *Hind*III generiert.
Die cDNA wurden mit dem Restriktionsenzym *Hind*III verdaut und über die Restriktionsschnittstellen *Ehe*l und *Hind*III in den Expressionsvektor pProExHT (Invitrogen, Carlsbad, USA) ligiert, und anschließend vollständig sequenziert.

### Beispiel 3: Gentechnische Konstruktion von Phl p 1 NoCys Δ1-6, 115-119, 213-220

Die DNA-Sequenz kodierend für die Deletionsvariante Phl p 1 NoCys Δ1-6, 115-119, 213-220 (SEQ ID NO 5 und 6) wurde mittels PCR unter Einsatz von spezifisch um den zu deletierenden Sequenzbereich verkürzten Oligonukleotiden in drei Schritten generiert.

Schritt 1 - Herstellung des N-terminalen DNA-Fragments "Phl p 1 NoCys Δ1-6 (bp 1-300)": PCR der cDNA von Phl p 1 NoCys mit den Primern P22-63 (Δ1-18) und P 250-318.

Schritt 2 - Herstellung des C-terminalen DNA-Fragments "Phl p 1 NoCys Δ115-119, 213-220 (bp 283-663)": PCR von Phl p 1 NoCys mit den Primern P 301-384 (Δ343-357) und P 613-720 (Δ637-660) *Hind*III.

Schritt 3 - Herstellung der vollständigen DNA kodierend für Phl p 1 NoCys Δ1-6, 115-119, 213-220 :
PCR der überlappenden Fragmente "Phl p 1 NoCys Δ 1-6 (bp 1-300)" und "Phl p 1 NoCys Δ115-119, 213-220 (bp 283-663)" mit den Primem P22-63 (Δ1-18) und P 703-720 *Hind*III.

Die für Phl p 1 NoCys Δ1-6, 115-119, 213-220 kodierenden DNA wurde mit dem Restriktionsenzym *Hind*III verdaut und über die Restriktionsschnittstellen Ehel und *Hind*III in den Expressionsvektor pProExHT (Invitrogen, Carlsbad, USA) ligiert, und anschließend vollständig sequenziert.

Die DNA der Varianten Phl p 1 NoCys Δ1-6 (SEQ ID NO 5 und 6), Phl p 1 NoCys Δ1-30 (SEQ ID NO 7 und 8), Phl p 1 NoCys Δ92-104 (SEQ ID NO 9 und 10), Phl p 1 NoCys Δ115-119 (SEQ ID NO 11 und 12), Phl p 1 NoCys Δ175-185 (SEQ ID NO 13 und 14) wurden in entsprechender Weise hergestellt, kloniert und sequenziert.

## Patentansprüche

1. Varianten der Gruppe 1-Allergene der *Poaceae*, welche durch ein gegenüber dem bekannten Wildtyp-Allergen verringerte IgE-Reaktivität sowie eine weitgehend erhaltene Reaktivität der T-Lymphozyten **gekennzeichnet** sind.

2. Allergenvarianten gemäß Anspruch 1 ausgewählt aus einer Gruppe bestehend aus *Phleum pratense, Lolium perenne, Poa pratensis, Holcus lanatus, Cynodon dactylon, Oryza sativa* und *Phalaris aquatica.*

3. Allergenvarianten gemäß einem oder mehreren der Ansprüche 1 bis 2, **dadurch gekennzeichnet, dass** die Cysteine der dem reifen Phl p 1 Protein entsprechenden Aminosäurepositionen 41, 57, 69, 72, 77, 83 und 139 fehlen.

4. Allergenvarianten gemäß einem oder mehreren der Ansprüche 1 bis 2, **dadurch gekennzeichnet, dass** die Cysteine der dem reifen Phl p 1 Protein entsprechenden Aminosäurepositionen 41, 57, 69, 72, 77, 83 und 139 durch eine andere Aminosäure substituiert werden.

5. Allergenvarianten gemäß Anspruch 4, **dadurch gekennzeichnet, dass** die Cysteine der dem reifen Phl p 1 Protein entsprechenden Aminosäurepositionen 41, 57, 69, 72, 77, 83 und 139 durch Serin substituiert werden.

6. Allergenvarianten, ausgewählt aus einer Gruppe bestehend aus dem reifen Phl p 1, Poa p 1, Hol p 1, Lol p 1 oder Pha a 1, bei denen die Sequenzveränderungen gemäß Anspruch 5 vorliegen.

7. Phl p 1-Variante gemäß Anspruch 6, **dadurch gekennzeichnet, dass** die Cysteine 41, 57, 69, 72, 77, 83 und 139 des reifen Proteins durch Serin substituiert werden (Variante Phl p 1 NoCys gemäß SEQ ID NO 4).

8. Allergenvarianten gemäß einem oder mehreren der Ansprüche 1-7, **dadurch gekennzeichnet, daß** mindestens ein Bereich oder eine Kombination von Bereichen, die den Aminosäuren 1-6, 1-30, 92-104, 115-119, 175-185 und 213-220 der Primärsequenz des reifen Phl p 1 Proteins entsprechen, gegenüber dem Wildtyp-Allergen fehlen.

9. Allergenvarianten gemäß einem oder mehreren der Ansprüche 1-8, bei denen die Aminosäuren 213-220 entsprechend der reifen Phl p 1-Sequenz fehlen.

10. Allergenvarianten, ausgewählt aus einer Gruppe bestehend aus dem reifen Phl p 1, Poa p 1, Hol p 1, Lol p 1 oder Pha a 1, bei denen die Sequenzveränderungen gemäß Anspruch 8 oder 9 vorliegen.

11. Phl p 1 -Variante gemäß Anspruch 10, bei der die Cysteine 41, 57, 69, 72, 77, 83 und 139 des reifen Proteins durch Serin ersetzt werden und Aminosäuren 213-220 fehlen (Variante Phl p 1 NoCys Δ213-220 gemäß SEQ ID NO 16).

12. Allergenvarianten gemäß Anspruch 8, bei denen die Aminosäuren 1-6, 115-119 und 213-220 entsprechend der reifen Phl p 1-Sequenz fehlen.

13. Allergenvarianten, ausgewählt aus einer Gruppe bestehend aus dem reifen Phl p 1, Poa p 1, Hol p 1, Lol p 1 oder Pha a 1, bei denen die Sequenzveränderungen gemäß Anspruch 12 vorliegen.

14. Phl p 1-Variante gemäß Anspruch 13 , bei der die Cysteine 41, 57, 69, 72, 77, 83 und 139 des reifen Proteins durch Serin ersetzt werden und Aminosäuren 1-6, 115-119 und 213-220 fehlen (Variante Phl p 1 NoCys Δ1-6, 115-119 und 213-220 gemäß SEQ ID NO 18).

15. Allergenvarianten gemäß Anspruch 1-14, **dadurch gekennzeichnet, daß** sie durch gentechnische Methoden rekombinant erhalten wurden.

16. DNA-Moleküle, kodierend für Allergenvarianten gemäß einem oder mehrerer der Ansprüche 1-15.

17. Rekombinanter Expressionsvektor, enthaltend ein DNA-Molekül gemäß Anspruch 16, funktionell verbunden mit einer ExpressionsKontrollsequenz.

18. Wirtsorganismus, transformiert mit einem DNA-Molekül gemäß Anspruch 16 oder einem Expressionsvektor gemäß Anspruch 15.

19. Verfahren zur Herstellung einer Allergenvariante gemäß einem oder mehrerer der Ansprüche 1-15 durch Kultivierung eines Wirtsorganismus gemäß Anspruch 18 und Gewinnung der entsprechenden Allergenvariante aus der Kultur.

20. Allergenvariante gemäß einem oder mehrerer der Ansprüche 1-15 als Arzneimittel.

21. Pharmazeutische Zubereitung, enthaltend mindestens eine Allergenvariante entsprechend einem oder mehreren der Ansprüche 1-15 und gegebenenfalls weitere Wirk- und/oder Hilfsstoffe zur präventiven und therapeutischen Behandlung von Allergien an deren Auslösung Gruppe 1-Allergene der *Poaceae* Spezies beteiligt sind.

22. Verwendung mindestens einer Allergenvariante gemäß einem oder mehreren der Ansprüche 1-15 zur Herstellung eines Arzneimittels zur Prävention und Therapie von Allergien an deren Auslösung Gruppe 1-Allergene von Spezies der *Poaceae* beteiligt sind.

23. DNA-Molekül gemäß Anspruch 16 als Arzneimittel.

24. Rekombinanter Expressionsvektor gemäß Anspruch 17 als Arzneimittel.

25. Pharmazeutische Zubereitung, enthaltend mindestens ein DNA-Molekül gemäß Anspruch 16 oder mindestens ein Expressionsvektor gemäß Anspruch 24 und gegebenenfalls weitere Wirk- und/oder Hilfsstoffe zur immuntherapeutischen DNA-Vakzinierung von Patienten mit Allergien, an deren Auslösung Gruppe 1-Allergene der *Poaceae* beteiligt sind und/oder zur Prävention solcher Allergien.

26. Verwendung mindestens eines DNA-Moleküls gemäß Anspruch 16 oder mindestens eines Expressionsvektors gemäß Anspruch 24 zur Herstellung eines Arzneimittels zur immuntherapeutischen DNA-Vakzinierung von Patienten mit Allergien, an deren Auslösung Gruppe 1-Allergene der *Poaceae* beteiligt sind und/oder zur Prävention solcher Allergien.
